# EUROPEAN PATENT APPLICATION

(11) **EP 1 666 018 A1**
(43) Date of publication of application: **07.06.2006**
(21) Application number: 06002913.9
(22) Date of filing: 21.09.2000
(51) Int. Cl.: A61J 1/00, A61K 41/00, C02F 1/00

(54) **Container for electrically preparing injectable substances**

(30) Priority: 21.09.1999 US 155465 P
(62) Divisional of application: 00968383.0
(71) Applicant: Orton, Kevin R., San Clemente, CA 92673 (US)
(72) Inventor: Orton, Kevin R., San Clemente, CA 92673 (US)
(74) Representative: Fiener, Josef

(57) **Abstract**

A container has at least one wall. A reservoir is defined within the wall. The reservoir can be sealed. At least one electrode extends at least partially into the sealed reservoir. An electrical contact element is connected to the electrode to facilitate electrically coupling the signal generator or other electrical supply to the electrode.

## Description

### BACKGROUND OF THE INVENTION

### Field of the Invention

The present invention generally relates to devices used to prepare therapeutic substances. More specifically, the present invention relates to devices used to prepare and dispense electrically-activated therapeutic substances.

### Related Art

One type of biotherapy generally uses an electrolytic fluid that is treated with electrical currents prior to use. In some applications, the fluid is injected into the recipient. The application of the electrical current to the fluid generally is done with a power supply and a signal generator. The electrical current is designed to trigger or enhance the therapeutic effectiveness of the electrolytic fluid or it's components.

In general, the electrolytic fluid is placed in a beaker, which holds the fluid and two or more electrodes. A signal generator passes an electrical current through the fluid between the electrodes to condition the fluid. After application of the current, the fluid is filtered and sterilized as needed before being placed in a syringe for injection into the patient. The patient is usually a human or mammal.

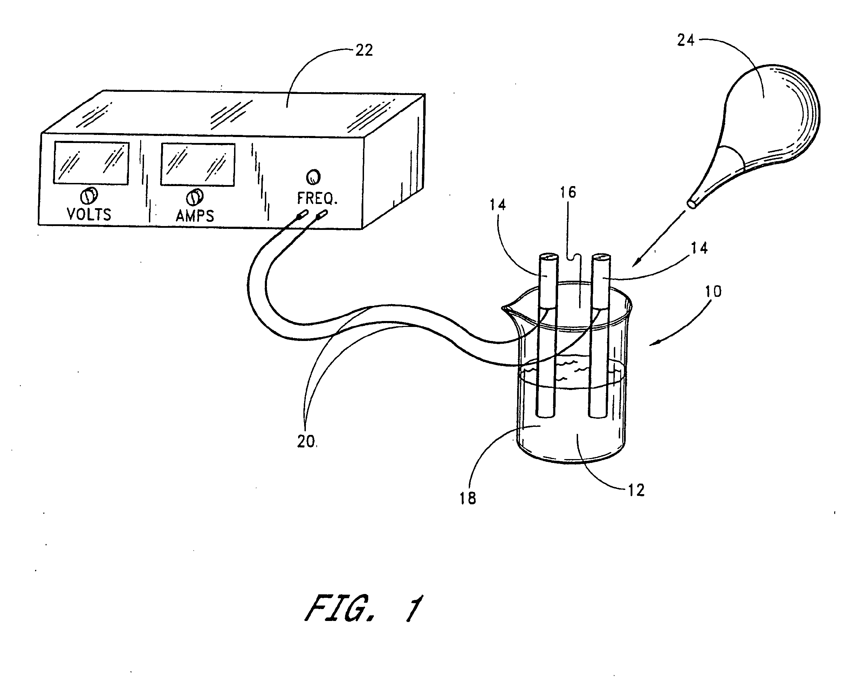

With reference now to Figure 1, conventional preparation equipment, which includes a beaker 10, is illustrated. The beaker 10 includes a reservoir area 12 for fluids, a set of electrodes 14 that extend into the reservoir area 12 and an opening area 16. Fluids 18 may be placed in the container 10 and an electric current can be applied to the fluids 18 through a set of wires 20 and the corresponding electrodes 14. The current can be supplied by a signal generator 22. When the application of electric current is completed, a utensil 24, such as a syringe, a tube, a squeeze bulb, a pipette, or any other suitable utensil, can be used to remove the electrically prepared fluid from the container 10. The fluid then is prepared for injection.

This process for preparing the active fluid has several disadvantages. One disadvantage is that the fluid must be handled quite a bit, such as when it is transferred between generally open containers to prepare it for use and to prepare it for administration. Additionally, the electrodes and the activating container must be cleaned and sterilized before each use, which activities are time consuming and require specialized equipment.

Therefore, a simpler, easier method of electrically preparing substances for injection is desired.

### SUMMARY OF THE INVENTION

Accordingly, a container has been developed for preparing and administering electrically prepared substances used by injection without requiring a transfer of the substances from one container to another container.

One aspect of the present invention involves a container adapted to hold a fluid substance. The container comprises at least one wall and a reservoir defined at least in part by the wall. At least two electrically conductive members extend into the reservoir. The electrically conductive members do not contact one another within the reservoir. The electrically conductive members are in electrical communication with respective contact elements disposed outside of said reservoir. A sealing portion cooperates with the outside surface to enclose the reservoir.

Another aspect of the present invention involves a method of preparing an electrically-activated injectable grade substance. The method comprises providing a container with a reservoir and at least one electrode extending into the container, at least partially filling the reservoir with an injectable grade substance, sealing the electrode and the substance in the reservoir, and applying an electrical signal to the electrode.

A further aspect of the present invention involves a flexible container adapted to hold a fluid. The container comprises a flexible wall that at least partially defines a reservoir. At least two electrically conductive electrodes are disposed at least partially in the reservoir. A gap is defined between the electrodes and the electrodes each have a portion exposed outside of the container when the container is closed.

### BRIEF DESCRIPTION OF THE DRAWINGS

These and other features, aspects and advantages of the present invention will now be described with reference to the drawings of a couple of preferred embodiments, which embodiments are intended to illustrate and not to limit the invention, and in which figures:
Figure 1 is a schematic illustration of traditional containers used in preparing an electrically prepared fluid;
Figure 2 is a side elevation view of a container constructed according to certain features, aspects and advantages of the present invention;
Figure 3 is an enlarged view of a seal of the container of Figure 2; and
Figure 4 is a schematic view of another container, which is used for intravenous feed and which also is constructed in accordance with certain features, aspects and advantages of the present invention.

### DETAILED DESCRIPTION OF THE PREFERRED EMBODIMENTS OF THE INVENTION

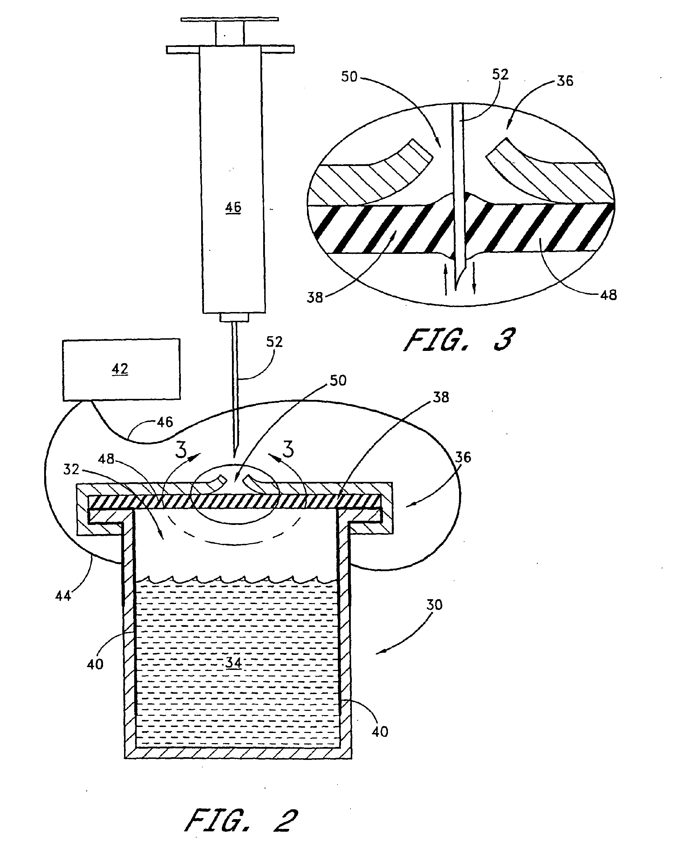

With reference now to Figure 2, a container having certain features, aspects and advantages in accordance with the present invention is illustrated. The illustrated container 30 comprises a vial-type of container. Of course, other containers may benefit from various features, aspects and advantages of the present invention. Preferably, the container 30 is a relatively small size. For example, in one arrangement, the container 30 is designed to hold between about 1 milliliter and 1 liter of fluid. In other arrangements, the container 30 is of a standard vial size and proportion such that the container 30 can be handled with exiting automated filling and assembly equipment. In some particularly advantageous arrangements, the container 30 is sized to accommodate a single dosage of electrically-prepared fluid.

With continued reference to Figure 2, a reservoir area 32 is defined within the container 30. In the illustrated arrangement, the container 30 accommodates any suitable fluids 34 and the reservoir 32 can be closed with a lid 36. A seal 38 preferably is interposed between a portion of the lid 36 and a portion of the container 30. The seal can be arranged in the manner described in my co-pending application entitled Vial With Vent Design, which was filed on the same date as this disclosure, which is hereby expressly incorporated by reference. In any event, the lid 36 desirably is tightly sealed to the container 30 and is configured to resist opening. In one arrangement, the lid 36 is designed to provide evidence of tampering after the lid 36 is secured to the container 30. Of course, the sealing construction, the tamper resistant construction and the tamper evidencing construction can be of any suitable configuration and many such constructions are well known.

Advantageously, electrically conductive electrodes 40 preferably are at least partially disposed within the reservoir area 32. In one arrangement, the electrodes 40 are directly secured to an inner surface of the container 30. The electrodes 40 are positioned such that they are in direct contact with at least a portion of the fluid 34 disposed within the container 30. While the illustrated electrodes 40 are placed on the sides of the container 30, the electrodes 40 can be placed on the ends of the container, wound in a circular or mounted in any other fashion while maintaining contact with the fluid 34 disposed within the container 30. In some arrangements, the electrodes 40 can be centrally disposed within the reservoir area 32.

With continued reference to Figure 2, the electrodes 40 can be positioned on opposing inner surfaces of the container 30. In is anticipated that the electrodes 40 can be placed in any number of different locations in the container 40. By positioning the electrodes 40 on opposing inner surfaces of the container 30, the separation of the electrodes 40 can be maximized. Of course, if-a non-round container is used, the electrodes 40 can be positioned is the corners, on opposing surfaces or in any other suitable location.

The electrodes 40 preferably extend outside of the reservoir area 32. In one arrangement, the electrodes 40 do not extend outside of the reservoir area 32 but are in electrical communication with connectors that are positioned outside of the sealed container 30. In another arrangement, the container 30 can be configured with only one electrode 40. With one electrode 40, the container 30 may be formed from an electrically conductive material or may comprise an electrically conductive member with the whole container 30 forming an electrode 40. In such a case, the signal generator that connects to the electrode 40 may be set to supply radio-frequency electrical signals to condition the substance in the container 30. In another arrangement, the electrode 40 can be configured so that the container 30 may comprise an electrically conductive material to form the first electrode 40 and an insulating portion and a second electrode 40 can be positioned inside the container 30. The electric current then flows from the electrode 40 inside the container 30, through the contents of the container 30 and to the conductive portion of the container 30.

The electrodes 40 preferably are formed of gold, titanium or other suitable metals or conductive elements. The electrodes 40 can be formed with electrodeposits of vapors, by electroplating, with metal foil tape or by any other suitable techniques. Preferably, the electrodes 40 are thin enough to not interfere with the seal 38 that is disposed between the lid 36 and the container 30. Of course, the electrodes 40 can be enlarged if an electrical connection can be established in other methods. For instance, the electrodes 40 can extend through at least a portion of the lid 36. In one presently preferred arrangement, the electrodes 40 generally are about 44 mm wide and about .30 mm thick. The exact sizing and configuration of the electrodes 40 can vary; however, the electrodes 40 preferably have a resistance below approximately 5000 ohms per square centimeter, and insulating portions disposed between the electrodes 40 should provide approximately 100 ohms or more resistance between the electrodes 40 to avoid shorting away excessive current.

In the illustrated arrangement, the seal 38 is configured in such a manner to provide a substantially contamination resistant seal between the contents of the container 30 and the exterior of the container 30. In addition, the electrodes 40 extend from the interior of the container 30, past the seal 38 to the outside of the container 30, where clip leads or other suitable electrical connections may be made.

The container 30 can be filled under controlled conditions using suitable processes. In one arrangement, the container 30 is filled with a sterile, injectable fluid 34 prior to the lid 36 being secured in position on the container 30. The processes advantageously produce a filled container 30 that has sterilized and relatively particulate free contents. In some applications, it is particularly important that the container 30 be free of even sterile particulate matter, in order to avoid an adverse reaction in the patient.

Prior to administration, a current can be applied to the container 30. In the illustrated arrangement, a signal generator 42 is used to supply the current. Various types of electrical signal generators 42 may be used to provide the electric signals. Genetronics, Inc., of San Diego, CA makes several bioelectric signal-generating apparatuses that can be used, for instance. The current preferably is applied for a preset period of time before any activated fluid 34 is withdrawn from the container 30.

The current travels from one wire 44 of the signal generator 42 to a clip (not shown). At the clip, the current is transferred to an exterior portion of one electrode 40. Of course, other electrical connections can result in the current passing through an intermediate member before passing to the electrode 40. In addition, various electrical connectors, plugs, or sockets, clip leads or other fittings may be used to provide electrical connection between the external portion of the electrode 40 and the electrical signal generator 42**.**

Once the current has flowed into the electrode 40, the current passes through the contents 34 of the container 30 to the second electrode 40. The current then is returned to the signal generator 42 or to a grounded connection by passing through a second wire 46. Additional electrodes (i.e., more than 2) can be used in some applications.

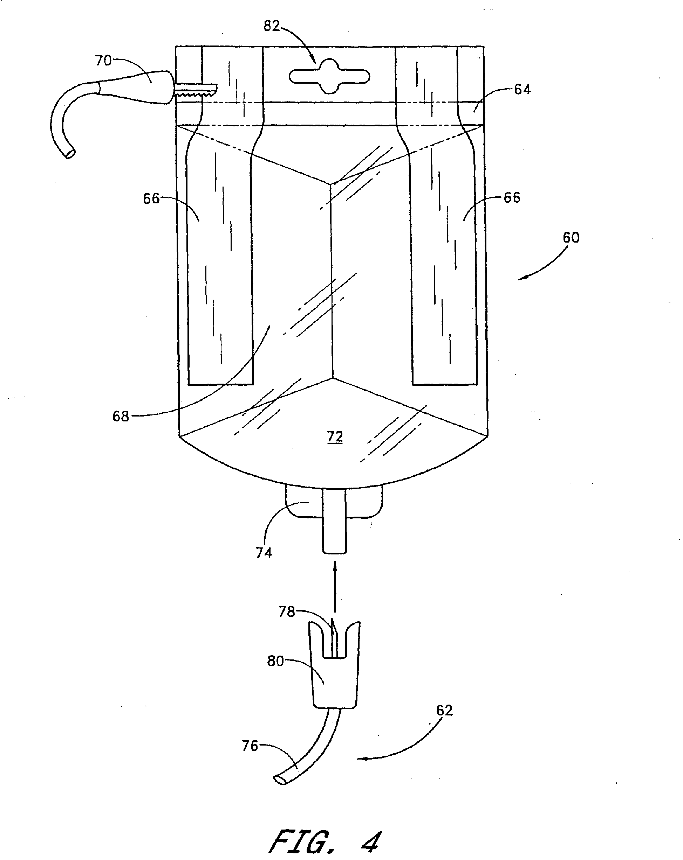

In the illustrated arrangement, the seal 38 comprises a barrier member 48 that allows the contents of the container 34 to be directly transferred to an applicator 46, such as a syringe, a catheter, a hypodermic needle, or any other suitable device, in a sterile manner. The member 48 can be any suitable septa. In some applications, the barrier member 48 comprises a soft type of elastomeric or rubber type material that is incorporated into a part of the container 30 or lid 36. For instance, the member 48, in combination with an access opening 50 formed in the lid 36, can provide a liquid and contamination tight seal while allowing a hypodermic needle 52 to temporarily pierce the seal 38 (see Figure 4). The needle 52 then can extract the electrically prepared fluid 34 from the container. Upon withdrawal of the needle 52, the barrier member 48 preferably reseals the opening through which the needle 52 was inserted. Thus, the barrier member 48 maintains a relatively contamination resistant seal for the contents of the container 30 while the syringe or other device is withdrawing activated fluids 34. Any suitable barrier member construction can be used.

With reference now to Figure 4, another arrangement of the container 60 is illustrated therein. As illustrated, the container 60 can be formed of a flexible vinyl-type material. In the illustrated configuration, the container 60 is a bag; however, other flexible designs (i.e., pouches, envelopes, etc.) also can be used. The illustrated bag 60 is configured and used to supply an intravenous feed 62. In addition, in the illustrated arrangement, the bag 60 is heat-sealed 44 at a seal line 64 after the fluids have been added. Preferably, one lip of the bag 60 is shorter than the other lip such that the longer lip extends a greater distance above the seal line 64 than the shorter lip for reasons that will become apparent. As will be recognized, no separate lid is needed in this arrangement.

With continued reference to Figure 4, a set of electrodes 66 preferably are at least partially disposed within the bag 60. In the illustrated arrangement, the electrodes 66 advantageously are formed on the interior walls of the bag 60. It should be recognized that the electrodes can be integrally formed with the walls 68 of the bag 60 in some applications such that the electrodes 66 might be placed within electrical communication with the contents of the bag 60 while also including a portion of the electrode 66 which is exposed to the exterior of the bag. In the illustrated arrangement, a portion of each electrode 66 is in electrical contact with the contents of the bag 60 and another portion of each electrode 66 passes through the seal line 64 of the bag 60 to the exterior of the bag 60. An electrical connection 70, such as a clip that is used in the illustrated arrangement, can be attached to the portion of the electrode 66 that is outside of the bag 60.

The electrodes 66 in the illustrated arrangement are positioned along opposing portions of the bag 60. The electrodes 66 can be of any suitable size and configuration. Preferably, the electrodes 66 are generally rectangular in shape. In addition, the electrodes 66 can be formed by various techniques. One such technique is to use a metalized foil strip laminated on to a vinyl substrate. The laminated metal then is incorporated into the construction of the bag 60.

A portion of the container 60, generally at a lower end 72 in the illustrated arrangement, preferably comprises one or more openings or fittings 74. The fittings 74 can be adapted to connect with a tube 76 or a hypodermic needle 78 such that the fluid contained within the bag 60 can be drawn outward in a controlled and sterile manner. In the illustrated configuration, the act of coupling the external tube 76 to the fitting 74 on the container 60 will break a seal portion on the container 60. For instance, the hypodermic needle 78 can be integrated to a fitting 80 that connects with the fitting 74 of the bag 60. The needle 78 can pierce the seal and extend into the fluid reservoir area. The fitting 80 may take various forms, with a standard luer type medical fitting providing acceptable results.

Preferably, the bag 60 is adapted to provide an intravenous feed supply. The vinyl portion can include any of a number of suitable mechanisms 82 for hanging the bag from a hook or other apparatus. In addition, the opening or fitting 74 can be located at the lower end 72 of the bag 60 to-provide gravity feed of the fluid from within the reservoir area. In some applications, a motorized pump or power feed mechanism can be used. Desirably, the bag 60 is configured so that at least a portion of the bag 60 is flexible and will collapse as the contents of the bag 60 are withdrawn, thereby preventing the entry of air into the bag 60 and maintaining a substantially controlled egress of fluid from the bag 60.

Several different types of operations can be performed on the contents of the container 30 or bag 60 fitted with the electrodes 40, 66. For instance, the resistance of the contents of the container 30 or bag 60 can be measured to determine the volume or composition of the contents. In some arrangements, the ionic properties of the fluid can be altered. In other arrangements, the ph level of the contents can be changed. Of course, other effects can be stimulated in suitable manners.

Preferably, in both arrangements, the container is filled and sterilized in a single location, using any suitable processes and any suitable automated equipment. The filled and sterilized container then can be transferred to the administration site. As will be appreciated, one aspect of the present invention provides that the substance within the container can be electrically prepared without need of removing it from the container prior to withdrawing at least a portion for injection. This aspect results in less transfer of the substance and improved sterility of the substance up until the moment of withdrawal for injection. In other words, there is minimal handling and minimal chance of contamination to the substance before, during, and after the time it is administered. Thus, this invention provides a construction whereby a sterile substance can be electrically prepared with minimal chance of contamination prior to injection. Thus, this device provides an easier to use, more sterile and more reliable method for administering electrically sensitive substances. It should be noted that, while the substance within the container may be injected, it need not be. The substance also may be applied topically or externally.

Although the present invention has been described in terms of certain preferred embodiments, other embodiments apparent to those of ordinary skill in the art also are within the scope of this invention. Thus, various changes and modifications may be made without departing from the spirit and scope of the invention. For instance, various components may be repositioned as desired. Moreover, not all of the features, aspects and advantages are necessarily required to practice the present invention. Accordingly, the scope of the present invention is intended to be defined only by the claims that follow.

## Claims

1. A container adapted to hold a liquid or fluid substance, the container comprising at least one wall, a reservoir defined at least in part by the wall, at least two electrically conductive members extending into the reservoir, said electrically conductive members not contacting one another within the reservoir, the electrically conductive members further being in electrical communication with respective contact elements disposed outside of said reservoir and a sealing portion cooperating with the outside surface to enclose the reservoir.

2. The container of Claim 1, wherein said container is at least partially filled with an injectable grade substance.

3. The container of Claim 1, wherein the sealing portion is a lid that is fitted with a soft pierceable member adapted to facilitate transfer of fluid material out of the container and into a hypodermic needle, in a contamination resistant manner.

4. The container of Claim 1 further comprising an access port.

5. The container of Claim 4, wherein said access port comprises a leur-type fitting.

6. The container of Claim 4, wherein said access port comprises a sealing portion adapted to reseal after penetration by a needle.

7. The container of Claim 1, wherein said electrodes are deposited on an inner surface of the at least one wall.

8. The container of Claim 1, wherein said electrodes are in direct contact with an inner surface of said at least one wall.

9. A container as in claim 1, wherein at least one wall of said container is formed of a flexible material.

10. A container as in claim 1, wherein said container is adapted to function as a medicament vial.

11. A method of preparing an electrically-activated injectable grade substance, the method comprising providing a container with a reservoir and at least one electrode extending from the outside into said reservoir, at least partially filling said reservoir with an injectable grade substance, sealing said inner electrode portion and said substance in said reservoir in a sterile manner, applying an electrical signal to the electrode.

12. The method of claim 9, where at least two electrodes extend into said container, with current passing between the two electrodes.

13. The method of Claim 9 or 10 further comprising removing at least a portion of the substance from the container after an electrical signal has been applied to the electrode.

14. The method of Claim 11 further comprising injecting at least a portion of the substance that was removed from the container.

15. A flexible container adapted to hold a fluid, the container comprising a wall that at least partially defines a reservoir, at least two electrically conductive electrodes being disposed at least partially in the reservoir, a gap being defined between the electrodes and the electrodes each having a portion exposed outside of the container when the container is closed.

16. The container of Claim 15, wherein the reservoir is at least partially filled with an injectable grade substance.

17. The container of Claim 15 further comprising a seal line at which the container is sealed shut to enclose the reservoir.

18. The container of Claim 17, wherein the electrodes extend through the seal line such that the exposed portion of the electrodes is on one side of the seal line and the reservoir is one the other side of the seal line.

19. The container of Claim 15 further comprising a hanging mechanism.

20. The container of Claim 15 further comprising an access port.

21. The container of Claim 20, wherein said access port is disposed within a fitting.

22. The container of Claim 21, wherein the fitting is positioned on a lower portion of the container and the container further comprises a hanging mechanism disposed higher than the fitting.

23. The container of Claim 15, wherein the container is a bag and the bag is adapted for dispensing of intravenous fluids.
